# EUROPEAN PATENT APPLICATION

(11) **EP 4 470 536 A1**
(43) Date of publication of application: **04.12.2024**
(21) Application number: 23176125.5
(22) Date of filing: 30.05.2023
(51) Int. Cl.: A61K 31/405, A61P 19/08

(54) **COMPOUND FOR USE IN TREATING FIBRODYSPLASIA OSSIFICANS PROGRESSIVA (FOP)**

(71) Applicant: Queen's University At Kingston, Kingston, Ontario K7L 3N6 (CA); Admare Bioinnovations, Montreal Québec H4S 1Z9 (CA)
(72) Inventor: The designation of the inventor has not yet been filed
(74) Representative: Boult Wade Tennant LLP

(57) **Abstract**

The present invention relates to a compound for use in treating fibrodysplasia ossificans progressiva (FOP) in a human subject, wherein the compound is in accordance with the following formula: 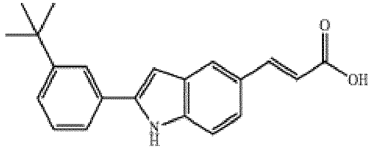 or a pharmaceutically acceptable salt thereof.

## Description

### Field of invention

The present invention relates to a compound for use in treating fibrodysplasia ossificans progressiva (FOP).

### Background of invention

FOP is a rare genetic disease in which heterotopic bone develops in muscle and soft tissue, leading to joint dysfunction and significant disability. The disease is characterised by episodic flare-ups in which patients experience painful, localised soft tissue inflammation, either spontaneously or in response to various triggers such as trauma, injury, immunisation or illness. These flare-ups lead to the formation of heterotopic endochondral bone in the muscle, joints, tendons, and ligaments. This, in turn, can result in severe joint restrictions and loss of mobility over time. FOP is progressive and many patients are wheelchair-bound by the third decade of life.

All FOP patients reported to date have been found to carry heterozygous gain-of-function mutations in the *ACVR1* gene. *ACVR1* encodes a bone morphogenetic protein (BMP) type 1 receptor, also known as the activin A Type 1 receptor. Aberrant signalling through this receptor leads to abnormal activation of the pSMAD 1/5/8 pathway and triggers the formation of bone outside of the skeleton. This bone formation is known as heterotopic ossification. A review of FOP and existing treatment strategies for FOP is provided in Wentworth et al, Therapeutic advances for blocking heterotopic ossification, Br J Clin Pharmacol, 2019, 85, 1180-1187. A review of the involvement of BMP/ACVR1 signalling is provided in Towler, O. W. and E. M. Shore. BMP signaling and skeletal development in fibrodysplasia ossificans progressiva (FOP), Dev Dyn, 2022, 251(1): 164-177.

Currently, there are no curative therapies for FOP. Corticosteroids and non-steroidal antiinflammatory drugs (NSAIDs) are currently in use for managing acute-phase inflammation during the episodic flare-ups mentioned above, but do not prevent heterotopic ossification *per* se or mitigate the progressive nature of the disease. The development of drugs for treating the heterotopic ossification associated with FOP has proved challenging, not least because *ACVR1* is widely expressed in humans, and BMP and activin A signalling play critical roles in multiple tissue types. To date, the only approved drug for treating FOP is palovarotene, marketed under the trade name Sohonos^{™} by Ipsen. Palovarotene was approved by Health Canada in January 2022 for use in reducing the formation of heterotopic ossification in adults and children aged 8 years and above for females and 10 years and above for males with FOP. Palovarotene acts as a retinoic acid receptor gamma (RARy) agonist, able to downregulate BMP signalling in prechondrogenic cells by promoting degradation of BMP-pathway specific SMAD1/5/8, and thus redirecting prechondrogenic mesenchymal stem cells from an osteoblast fate to a non-osseous soft tissue fate, and thereby reducing heterotopic ossification.

However, the use of palovarotene in treating FOP brings significant side effects that limit its utility. First, palovarotene has been found to cause premature epiphyseal growth plate closure in young children, leading to an underdeveloped skeleton and growth defects. This explains the decision of Health Canada to specify minimum ages in the approved indication (which are the ages at which 90% of growth plate has closed). Indeed, the present inventors have found that palovarotene has no therapeutic window in a juvenile mouse model of FOP (that is, there is no dose at which the drug is effective without causing growth plate defects). Second, palovarotene can cause dry-eye disease. Third, the skin of palovarotene-treated patients can show signs of retinoid toxicity. At least some of the side effects of palovarotene may be linked with off-target interactions caused by the ubiquitous expression of RARs (the target for palovarotene and other retinoid agonists) across essentially all cell types.

Accordingly, it is one object of the present invention to provide a FOP therapy that has a broader therapeutic window than palovarotene.

It is an alternative and/or additional object of the present invention to provide a FOP therapy that leads to reduced toxicity and/or side effects compared with palovarotene and/or can be used in younger patient groups.

It is an alternative and/or additional object of the present invention to provide a FOP therapy that has greater efficacy than palovarotene.

### Summary of invention

According to a first aspect, the present invention provides a compound for use in treating fibrodysplasia ossificans progressiva (FOP) in a human subject, wherein the compound is in accordance with the following formula: 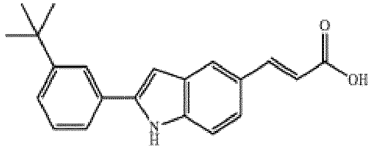 or a pharmaceutically acceptable salt thereof.

This first aspect also encompasses a method for treating fibrodysplasia ossificans progressiva (FOP) in a human subject, the method comprising administering to the subject a compound in accordance with the following formula: 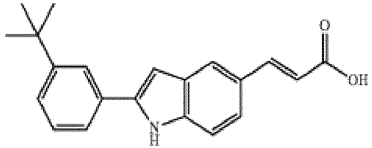 or a pharmaceutically acceptable salt thereof.

This first aspect also encompasses use of a compound in accordance with the following formula: 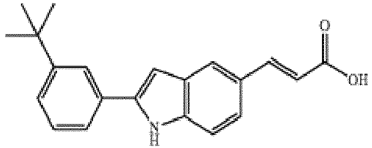 or a pharmaceutically acceptable salt thereof, in the manufacture of a medicament for the treatment of fibrodysplasia ossificans progressiva (FOP) in a human subject.

Based on preclinical studies, the present inventors have found that the compound may be used to treat FOP and advantageously may be more efficacious and/or exhibit reduced side effects and/or have a broader therapeutic window than palovarotene. In particular, the compound may be used to treat a broader patient population than palovarotene, including younger children.

### Brief description of the figures

The present invention will now be described in relation to the following non-limiting figures:

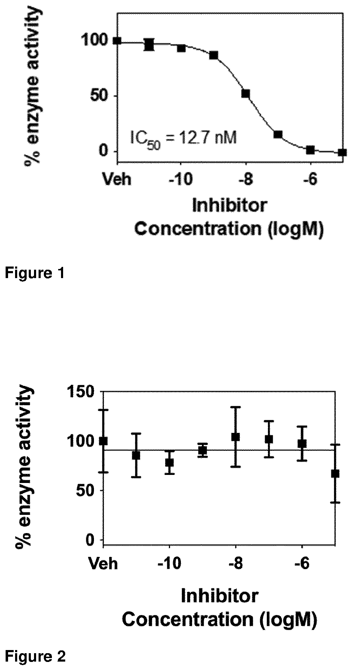

**Figure 1** shows a dose-response curve showing the effect of compound 1 on inhibiting all trans-RA metabolism in the HeLa-CYP26B1 cell-based inhibitor assay of Example 2. The y-axis denotes the amount of all trans-RA metabolites measured in the assay as a percentage relative to a vehicle comprising no test compound, while the x-axis denotes the log of the molar concentration (M) of the test compound. The error bars denote standard deviation.
**Figure 2** shows a dose-response curve showing the effect of compound 1 on inhibiting all trans-RA metabolism in the HeLa-CYP26A1 cell-based inhibitor assay of Example 2. The y-axis denotes the amount of all trans-RA metabolites measured in the assay as a percentage relative to a vehicle comprising no test compound, while the x-axis denotes the log of the molar concentration (M) of the test compound. The error bars denote standard deviation.

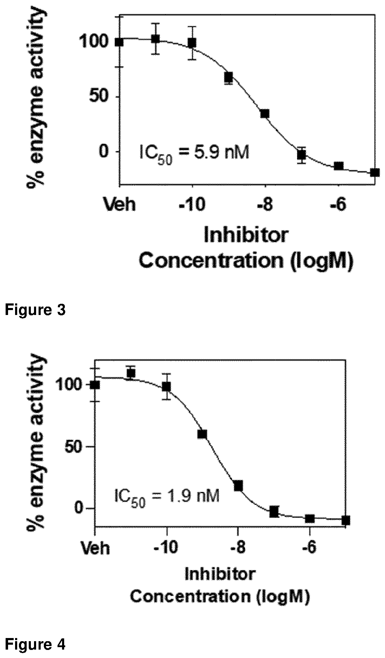

**Figure 3** shows a dose-response curve showing the effect of compound 1 on inhibiting all trans-RA metabolism in the microsome-based inhibitor assay of Example 3. The y-axis denotes the amount of all trans-RA metabolites measured in the assay as a percentage relative to a vehicle comprising no test compound, while the x-axis denotes the log of the molar concentration (M) of the test compound. The error bars denote standard deviation.
**Figure 4** shows a dose-response curve showing the effect of compound 1 on inhibiting all trans-RA metabolism in the U2OS cell-based inhibitor assay of Example 4. The y-axis denotes the amount of all trans-RA metabolites measured in the assay as a percentage relative to a vehicle comprising no test compound, while the x-axis denotes the log of the molar concentration (M) of the test compound. The error bars denote standard deviation.

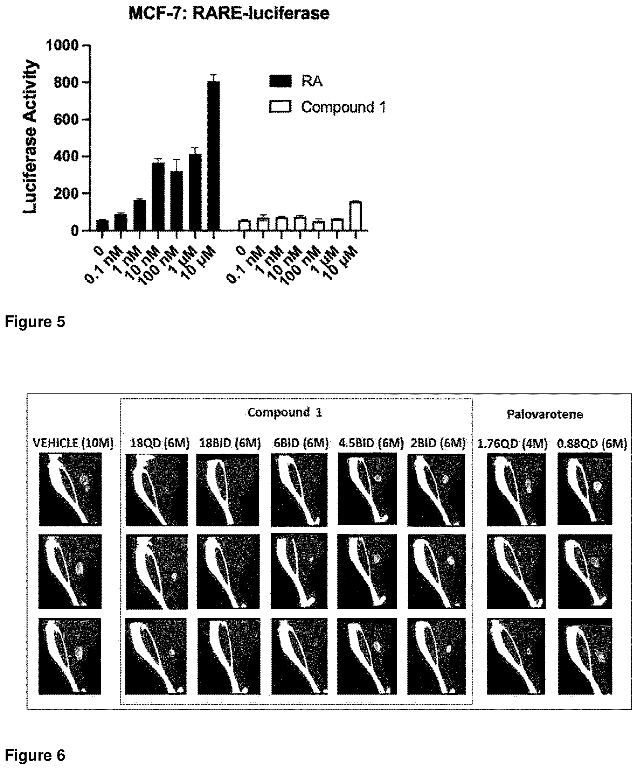

**Figure 5** shows a bar graph illustrating the effect of compound 1 (white bars) or RA (black bars) on inducing luciferase activity in the RAR transcriptional activation assay of Example 5. The y-axis denotes luciferase activity measured in the assay, while the x-axis denotes the concentration of the test compound. The error bars denote standard deviation.
**Figure 6** shows representative DICOM renderings obtained from micro-CT imaging of various treatment groups following surgical induction of HO and administration of compound 1, palovarotene or a control, as described in Example 6. Doses in mg/kg, treatment regimens (QD or BID), and number of animals examined are indicated. The highly enhancing areas seen within the gastrocnemius muscle represent ectopic bone formed at the site of collagen disk implantation.

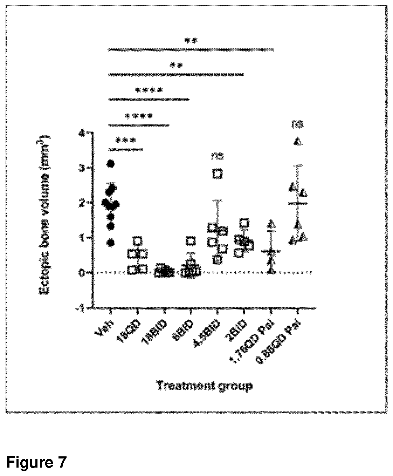

**Figure 7** shows a whisker plot summarizing the volume in mm³ (y-axis) of ectopic bone that was formed after 14 days of treatment for each group, indicated on the x-axis, as described in Example 7. "Veh" refers to vehicle, "Pal" refers to palovarotene and the other data points relate to compound 1. Each point represents one animal, with mean ± standard deviation. Statistical significance from the vehicle-treated group was calculated using an unpaired student's t-test (ns = not significant, * = p<0.05 ** = p<0.01, *** = p<0.001, **** = p,0.0001).

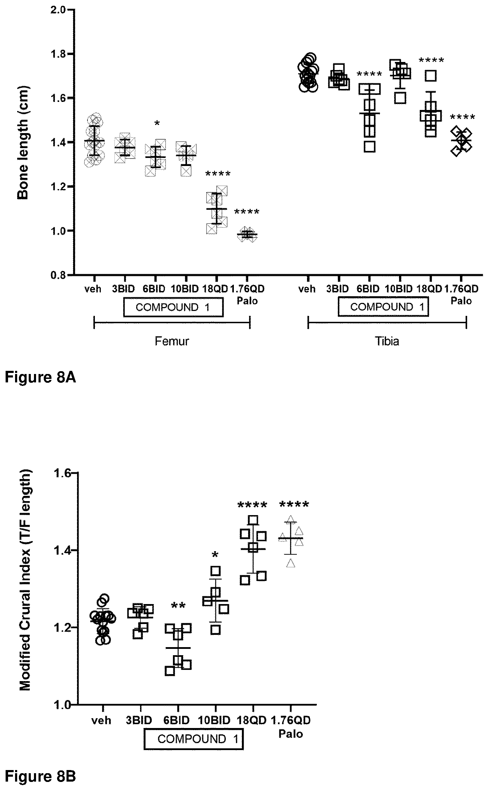

**Figure 8A** shows a whisker plot representing the lengths of tibiae and femurs in cm (y-axis) from animals treated with various doses of compound 1 versus palovarotene listed along the x-axis for 4 weeks over the juvenile period of bone development, as described in Example 8.
"veh" refers to vehicle, "Palo" refers to palovarotene and "1" refers to compound 1. Each point represents one measurement, with mean ± standard deviation. Statistical significance from vehicle-treated animals was calculated using an unpaired student's t-test (* = p<0.05 ** = p<0.01, *** = p<0.001, **** = p,0.0001).
**Figure 8B** shows a whisker plot representing modified crural index (the length of tibiae divided by the length of femurs) on the y-axis, calculated for animals treated with various doses of compound 1 versus palovarotene, outlined on the x-axis, for 4 weeks over the juvenile period of bone development, as described in Example 8. "veh" refers to vehicle, "Palo" refers to palovarotene and "1" refers to compound 1. Each point represents one measurement, with mean ± standard deviation. Statistical significance from vehicle-treated animals was calculated using an unpaired student's t-test (* = p<0.05, ** = p<0.01, *** = p<0.001, **** = p,0.0001).

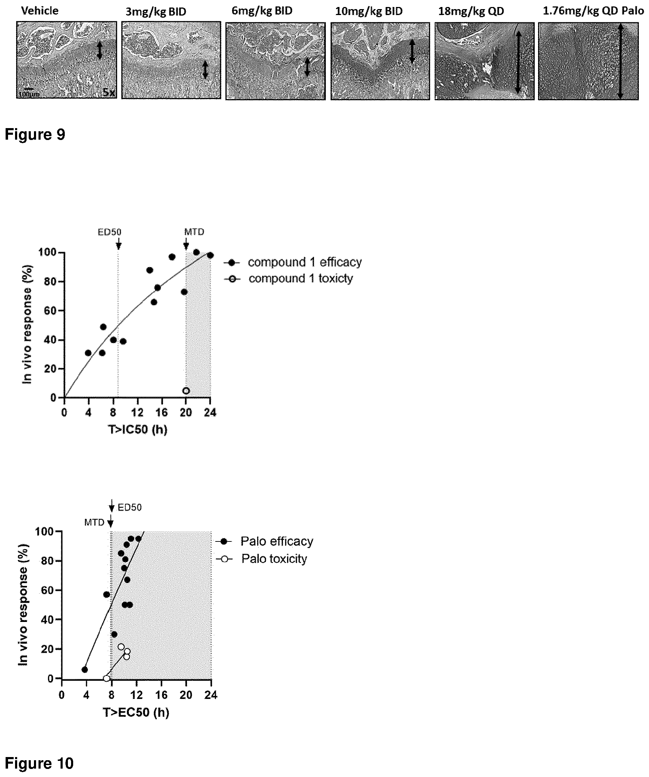

**Figure 9** shows representative sections from proximal tibial growth plates stained with Safranin O/Fast Green, as described in Example 8 ("Palo" refers to palovarotene, while the other images that are not the vehicle are for different doses of compound 1). The arrows indicate the epiphyseal cartilage of the growth plate, as shown by positive Safranin O staining, inclusive of the resting, proliferative, and hypertrophic zones for each image.
**Figure 10** shows plots representing individual data points of efficacy and toxicity (>5% increase in modified crural index) relative to time above EC50 for palovarotene ("Palo"), or IC50 for compound 1, illustrating the respective therapeutic windows for compound 1 and palovarotene, as described in Example 9.

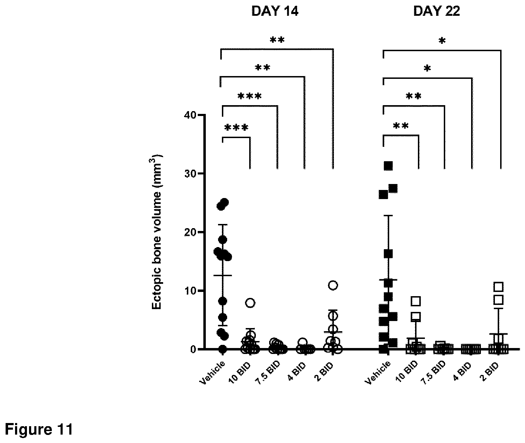

**Figure 11** shows a whisker plot of the volume (mm³) of total ectopic bone formed on the y-axis following pinch injury of *Acvr1*^{*(cR206H*/}*⁺⁾;Pdgfra-Cre+* animals after either 14 or 21 days of treatment with compound 1, as described in Example 10. Treatment groups are outlined on the x-axis (compound 1 at doses of 2 mg/kg, 4 mg/kg, 7.5 mg/kg and 10 mg/kg BID, and vehicle). Each point represents one measurement, with mean ± standard deviation. Statistical significance from vehicle-treated animals was calculated using an unpaired student's t-test (* = p<0.05, ** = p<0.01, *** = p<0.001, **** = p,0.0001).

### Detailed description

The present invention will now be described further. In the following passages different aspects/embodiments of the invention are defined in more detail. Each aspect/embodiment so defined may be combined with any other aspect/embodiment or aspects/embodiments unless clearly indicated to the contrary. In particular, any feature indicated as being preferred or advantageous may be combined with any other feature or features indicated as being preferred or advantageous.

### A. Definitions and abbreviations

Unless defined otherwise, all technical and scientific terms used herein have the same meaning as is commonly understood by one skilled in the art in the technical field of the invention.

**ACVR1** - As used herein, the term "ACVR1" refers to Activin A receptor, type I. Details of the human homolog of ACVR1 (including the protein sequence) are deposited in the Uniprot database as Q04771 - ACVR1_HUMAN.

**ALDH1A2** - As used herein, the term "ALDH1A2" refers to aldehyde dehydrogenase 1 family member A2, an enzyme that catalyses the synthesis of retinoic acid from retinaldehyde. Details of the human homolog of ALDH1A2 (including the protein sequence) are deposited in the Uniprot database as O94788 - AL1A2_HUMAN.

**BID** - As used herein, the term "BID" refers to twice-daily administration.

**BMP** - As used herein, the term "BMP" refers to Bone Morphogenetic Protein.

**CD-1** - As used herein, the term "CD-1" refers to the outbred albino CD-1^{®} IGS multi-purpose mouse strain.

**CT** - As used herein, the term "CT" refers to computed tomography.

**CYP26** - As used herein, the term "CYP26" refers to cytochrome P450 family 26. This family of cytochrome P450 enzymes, which comprises CYP26A1 and CYP26B1, has a role in the metabolism of retinoic acid.

**CYP26A1** - As used herein, the term "CYP26A1" refers to cytochrome P450 family 26 subfamily A member 1. Details of the human homolog of CYP26A1 (including the protein sequence) are deposited in the Uniprot database as O43174 - CP26A_HUMAN.

**CYP26B1** - As used herein, the term "CYP26B1" refers to cytochrome P450 family 26 subfamily B member 1. Details of the human homolog of CYP26B1 (including the protein sequence) are deposited in the Uniprot database as Q9NR63 - CP26B_HUMAN.

**CYP26B1 inhibitor** - As used herein, the term "CYP26B1 inhibitor" refers to any agent, compound or molecule capable of inhibiting the activity of CYP26B1 by binding to CYP26B1, particularly the retinoic acid metabolising activity of CYP26B1.

**DICOM** - As used herein, the term "DICOM" refers to Digital Imaging and Communications in Medicine^{®}, which is the international standard for files consisting of medical images and related information.

**DMSO** - As used herein, the term "DMSO" refers to dimethyl sulfoxide.

**EC50** - As used herein, the term "EC50" refers to the concentration of drug required for 50% maximal effective activity of the drug.

**ED50** - As used herein, the term "ED50" refers to the effective dose for 50% reduction in ectopic bone formation.

**F** - As used herein, the term "F" refers to female.

**FBS** - As used herein, the term "FBS" refers to fetal bovine serum.

**FOP** - As used herein, the term "FOP" refers to the rare genetic condition, Fibrodysplasia Ossificans Progressiva.

**FVB** - As used herein, the term "FVB" refers to the inbred albino mouse strain (strain code 207) named after its susceptibility to Friend leukemia virus B. It is available from Charles River Laboratories (bred in Raleigh, NC, USA).

**HO** - As used herein, the term "HO" refers to heterotopic ossification. Heterotopic ossification is the term used to describe the formation of ectopic bone in soft tissue.

**IC50** - As used herein, the term "IC50" refers to the concentration of drug required for 50% inhibition of CYP26B1 activity.

**IFOPA** - As used herein, the term "IFOPA" refers to the International Fibrodysplasia Ossificans Progressiva Association (North Kansas City, MO, USA).

**kVp** - As used herein, the term "kVp" refers to kilovoltage peak.

**M** - As used herein, the term "M" refers to male.

**MEM** - As used herein, "MEM" refers to Minimum Essential Medium.

**mg/kg** - As used herein, the term "mg/kg" refers to milligrams of compound per kilogram body weight.

**ms** - As used herein, the term "ms" refers to millisecond.

**MTD** - As used herein, the term "MTD" refers to the maximum tolerated dose

**P** - As used herein, the term "P" refers to postnatal day.

**Palovarotene** - As used herein, the term "palovarotene" refers to the compound having the following chemical structure:

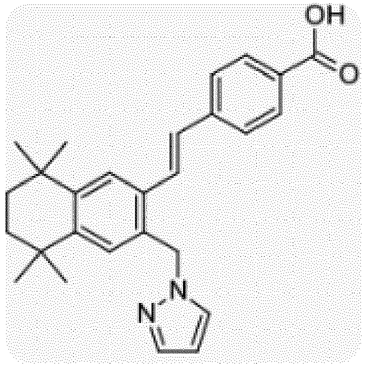

**PDGFRA** - As used herein, the term "PDGFRA" refers to platelet-derived growth factor receptor alpha. Details of the human homolog of PDGFRA (including the protein sequence) are deposited in the Uniprot database as P16234 - PDGFRA_HUMAN.

**Pediatric** - As used herein, the term "pediatric" refers to a patient who is under the age of 18 years at the time of treatment. The term "pediatric" can be further divided into various subpopulations including: neonates (from birth through the first 28 days of life); infants (29 days of age to less than two years of age); children (two years of age to less than 12 years of age); and adolescents (12 years of age through 17 years of age (up to, but not including, the eighteenth birthday).

**Pharmaceutically acceptable salt** - As used herein, the term "pharmaceutically acceptable salt" refers to ionic compounds formed by the addition of an acid to a base, specifically such compounds that are considered in the art as being suitable for use in contact with a patient, for example *in vivo.* Pharmaceutically acceptable salts are generally chosen for their non-toxic, non-irritant characteristics.

Salts known in the art to be generally suitable for use in contact with a patient include salts derived from inorganic acids, organic acids, or alkali or alkaline earth metals. Further reference is made to the number of literature sources that survey suitable pharmaceutically acceptable salts, for example the handbook of pharmaceutical salts published by IUPAC.

**QD** - As used herein, the term "QD" refers to once-daily administration.

**RA** - As used herein, the term "RA" refers to retinoic acid.

**RAR** - As used herein, "RAR" refers to retinoic acid receptor, a type of nuclear receptor which can also act as a ligand-activated transcription factor that is activated by retinoic acid. RARs include alpha, beta and gamma subtypes.

**rhBMP2** - As used herein, the term "rhBMP2" refers to recombinant human Bone Morphogenetic Protein 2. Details of the human homolog of BMP2 (including the protein sequence) are deposited in the Uniprot database as P12643 - BMP2_HUMAN.

**µCT** or **micro-CT** - As used herein, the terms "µCT" or "micro-CT" refer to micro computed tomography.

**µA** - As used herein, the term "µA" refers to microampere.

**µg** - As used herein, the term "µg" refers to microgram.

**µm** - As used herein, the term "µm" refers to micrometer.

### B. Medical uses

The first aspect of the present invention relates to a compound for use in treating fibrodysplasia ossificans progressiva (FOP) in a human subject. The compound is in accordance with the following formula: 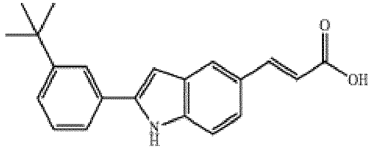 or is a pharmaceutically acceptable salt thereof.

This first aspect also encompasses a method for treating fibrodysplasia ossificans progressiva (FOP) in a human subject, the method comprising administering to the subject a compound in accordance with the following formula: 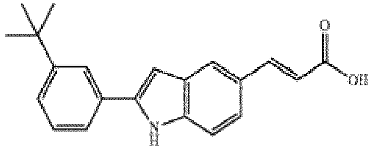 or a pharmaceutically acceptable salt thereof.

This first aspect also encompasses use of a compound in accordance with the following formula: 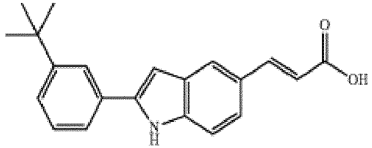 or a pharmaceutically acceptable salt thereof, in the manufacture of a medicament for the treatment of fibrodysplasia ossificans progressiva (FOP) in a human subject.

### Compound for use

The compound for use in accordance with the present invention is represented by the following formula:

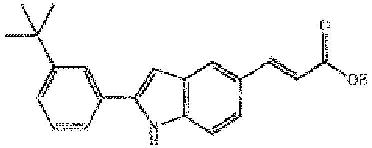

This compound is also referred to elsewhere herein as "compound 1". It is to be appreciated that each of the atoms forming part of the depicted compound may be the common isotope (e.g. ¹H or ¹²C) or an isotope other than the common isotope (e.g. ²H, ³H or ¹³C).

The invention also encompasses use of pharmaceutically acceptable salts of compound 1. The term "pharmaceutically acceptable salt" is defined herein elsewhere. The pharmaceutically acceptable salt may be synthesised from the compound shown above by conventional chemical methods. In some embodiments, the pharmaceutically acceptable salt is a metal salt. In some embodiments, the metal salt is derived from an alkali metal or an alkaline earth metal. In other embodiments, the pharmaceutically acceptable salt is an ammonium or alkylammonium salt. Other possible salts of carboxylic acids are known to those skilled in the art. In some embodiments, the compound for use is in accordance with the formula depicted above, i.e. is the free-carboxylic acid form, rather than a pharmaceutically acceptable salt thereof.

Based on preclinical studies, the present inventors have found that the compound described herein may be used to treat FOP. It is believed that the utility of the compound is linked to its ability to inhibit CYP26B1 and thereby inhibit the metabolism of retinoic acid, which is known to impact bone cell differentiation at different stages of development. In particular, the compound selectively inhibits CYP26B1 over CYP26A1. To the best of the inventors' knowledge, this is the first time that CYP26B1 inhibition has been identified as a strategy for treating FOP.

The compound for use in accordance with the invention may offer a number of advantages over palovarotene and other RAR agonists. First, the compound is demonstrated in the Examples of the present application to have a greater therapeutic window than palovarotene. In particular, there is a range of doses at which the compound is able to reduce heterotopic ossification in a mouse model without the juvenile growth plate toxicity that is associated with palovarotene. This, in turn, means that the compound may be used to treat younger patients with immature bone development, whereas palovarotene has been found to cause premature epiphyseal growth plate closure leading to an underdeveloped skeleton and growth defects.

Without wishing to be bound by theory, it is believed that the low propensity for off-target effects may be linked, at least in part, to the fact that fewer cell types express CYP26B1, whereas essentially all cell types express RARs. As such, the compound of the invention may be more targeted to the osteochondrogenic lineage than the RAR agonist palovarotene and therefore more selective towards sites of ectopic bone formation. Moreover, CYP26B1 inhibition as a means of reducing heterotopic ossification requires local retinoic acid production. This is usually provided by the activity of enzymes such as ALDH1A2, which converts retinaldehyde to retinoic acid in a localised, restricted manner. The requirement for local retinoic acid production further restricts the cell types affected by CYP26B1 inhibition, potentially further reducing the potential for off-target effects.

Furthermore, as noted above, the compound selectively inhibits CYP26B1 over CYP26A1. In particular, the compound does not inhibit CYP26A1 or inhibits CYP26A1 only minimally. This may help to avoid a significant increase in circulating (as opposed to localised) retinoic acid, reducing the compound's propensity for further off-target effects. For instance, the compound may avoid the retinoid toxicity manifestations that are observed in the skin of palovarotene-treated patients. The present inventors also believe that the compound of the invention is less likely to cause dry-eye disease than palovarotene, as observed in mouse models and palovarotene-treated patients.

The compound for use in accordance with the invention and its synthesis are disclosed in WO 2019/113693 A1. This document shows that the compound inhibits CYP26B1 *in vitro* and lists a number of possible therapeutic uses for the compounds disclosed therein. This document does not, however, disclose the treatment of genetic disorders of ectopic bone formation, let alone FOP specifically.

### Treating subjects with FOP

As noted above, all FOP patients reported to date have been found to carry heterozygous gain-of-function mutations in the *ACVR1* gene. In most cases, a single nucleotide transition (c.617G>A) causes a missense mutation in codon 206, resulting in substitution of arginine by histidine (R206H) in the intracellular glycine-serine (GS) domain of ACVR1 (Shore EM, Xu M, Feldman GJ, et al. A recurrent mutation in the BMP type I receptor ACVR1 causes inherited and sporadic fibrodysplasia ossificans progressiva, Nat Genet., 2006; 38(5):525-527.). It follows that in some embodiments, the human subject to be treated in accordance with the claims has a R206H mutation in the intracellular glycine-serine (GS) domain of human ACVR1.

In some embodiments, the human subject is a pediatric subject. In some embodiments, the human subject has an age of 17 years or less, or 16 years or less, or 14 years or less, or 12 years or less, or 10 years or less. In some embodiments, the human subject has an age of 4 years or more, or 5 years or more.

In some embodiments, the human subject is a male having an age of 9 years or less. In some embodiments, the human subject is a female having an age of 7 years or less. As explained above, the compound as described herein may be used to treat younger patient groups than palovarotene in view of its reduced potential for juvenile growth plate toxicity.

The compound as described herein is for use in treating fibrodysplasia ossificans progressiva (FOP). As explained elsewhere herein, FOP is a rare genetic disease characterised by heterotopic ossification (HO). HO is the term used to describe bone formation at extraskeletal or ectopic sites, particularly in soft tissue. This ectopic bone formation leads to joint dysfunction and significant disability.

FOP is characterised by episodic flare-ups in which patients develop painful, localised soft tissue inflammation. These flare-ups lead to HO of endochondral origin, predominantly at muscles, tendons, ligaments and connective tissue. This results in severe joint restrictions and loss of mobility over time.

The compound as described herein is for use in treating FOP, wherein "treating" may mean the alleviation or eradication of any symptoms associated with the disease such that the subject's suffering is reduced.

Treating FOP may comprise decreasing or preventing heterotopic ossification.

The flare-ups observed in subjects with FOP are typically triggered by some form of injury, ranging from significant trauma to more trivial events such as injections, muscle trauma from bumps, bruises and falls, and illness such as influenza-like illnesses. It follows that in certain embodiments, treating FOP comprises decreasing or preventing injury-induced heterotopic ossification wherein "injury" encompasses any form of trauma described above.

In certain preferred embodiments, treating FOP comprises decreasing or preventing the formation of heterotopic ossification lesions in the subject. As used herein, the term "heterotopic ossification lesion" is intended to mean the site or location of HO within the body. In some embodiments, the heterotopic ossification lesions comprise or consist of calcified soft tissue. The soft muscle tissue may comprise calcified muscle tissue.

As noted above, FOP is associated with HO of endochondral origin and as such, treating FOP may comprise decreasing or preventing endochondral ossification. Endochondral ossification may be defined as the process whereby mesenchymal cells form cartilage that proliferates and matures to be replaced eventually by osteoblasts leading to bone formation.

In some embodiments, the subject exhibits a decrease in the formation of heterotopic ossification lesions of at least 5%, at least 10%, at least 20%, at least 30%, at least 40%, at least 50%, at least 60%, at least 70%, at least 80% or at least 90% relative to a control. In certain preferred embodiments, the subject exhibits a decrease in the formation of heterotopic ossification lesions of at least 50%. These decreases may represent a reduction in the number of lesions that form over a certain period of time. Alternatively, these decreases may represent a reduction in the rate at which new lesions form. In certain preferred embodiments, the decreases represent a reduction in the volume of HO lesions that form over a certain period of time. The period of time may be from the time of first treatment with the compound to the end of the treatment period. The "control" against which the decrease is measured may be the same subject prior to treatment with the compound. Alternatively, the "control" may be an untreated subject against which the treated subject is compared.

As discussed elsewhere herein, the compound for use in accordance with the present invention exhibits a number of advantages as compared with the use of palovarotene in the treatment of FOP. These advantages stem, at least in part, from the inhibition of CYP26B1 as opposed to the RARy agonist activity of palovarotene. As reported herein, use of the compound as described herein is associated with reduced toxicity. It follows that treating subjects with FOP in accordance with the present claims may be associated with an absence or significant reduction in toxicity (as compared with use of palovarotene).

In certain embodiments, treating subjects with FOP in accordance with the present claims is not associated with a significant change in modified crural index (ratio of tibia length to femur length in the subject) as compared with a control. In preferred embodiments, the modified crural index does not increase by more than 5% as compared with a control, preferably not more than 2%, preferably not more than 1% as compared with a control. As explained, for example in Davenport, C. B. (1933). "The Crural Index." American Journal of Physical Anthropology 17(3): 333-353, the crural index is the ratio of tibia length (multiplied by 100) to femur length. The "modified" crural index is modified in the sense that the 100 multiplication factor is not applied to the tibia length. In other words, by modified crural index it is meant the ratio of tibia length to femur length. The control may be the modified crural index calculated for the subject prior to the treatment. Alternatively, the control may be a standardised or mean modified crural index for a population of individuals of a sex and age equivalent to the subject's sex and age.

In certain embodiments, reduced toxicity may manifest as a reduction in damage to or closure of growth plates. "Growth plates" are the areas located at the epiphysis of long bones which contribute new bone growth found in children and teens.

### Pharmaceutical compositions and routes of administration

In some embodiments, the compound is provided in a pharmaceutical composition comprising the compound and one or more pharmaceutically acceptable diluents, excipients or carriers.

Thus, in a further aspect, the present invention provides a pharmaceutical composition for use in treating fibrodysplasia ossificans progressiva (FOP) in a human subject, wherein the pharmaceutical composition comprises the compound in accordance with the following formula: 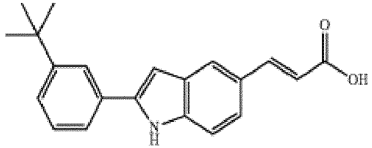 or a pharmaceutically acceptable salt thereof, and one or more pharmaceutically acceptable diluents, excipients or carriers.

Suitable pharmaceutically acceptable diluents, excipients and carriers are known by the person skilled in the art and include but are not limited to: fats, water, physiological saline, alcohol (e.g. ethanol), glycerol, polyols, aqueous glucose solution, extending agent, disintegrating agent, binder, lubricant, wetting agent, stabilizer, emulsifier, dispersant, preservative, sweetener, colorant, seasoning agent or aromatizer, concentrating agent, buffer substance, solvent or solubilizing agent, chemical for achieving storage effect, salt for modifying osmotic pressure, coating agent or antioxidant, saccharides such as lactose or glucose; starch of corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; alcohols such as stearyl alcohol or benzyl alcohol; synthetic cellulose derivatives such as methylcellulose, carboxymethylcellulose, ethylcellulose or hydroxypropylmethylcellulose; and other conventionally used additives such as gelatin, talc, plant oil and gum arabic.

The compound for use as described herein may be formulated for administration via any suitable route of administration including but not limited to enteral (oral and rectal) and parenteral routes of administration. Routes of parenteral administration may include but are not limited to sublingual administration, intravenous administration, subcutaneous administration, transdermal administration and intramuscular administration. For parenteral administration, for example intravenous, intramuscular or subcutaneous use, a sterile aqueous solution may be provided that may contain other substances including (but not limited to) salts and/or glucose to make the solution isotonic. For injectable solutions, excipients include, for example, water, alcohols, polyols, glycerine and vegetable oil.

In certain preferred embodiments, the compound is administered orally. For oral administration, the compound may be formulated as tablets, coated tablets, hard or soft gelatine capsules, solutions, emulsions, or suspensions. For the preparation of tablets, coated tablets or hard gelatine capsules, the compound for use in accordance with the present invention may be admixed with pharmaceutically inert, inorganic or organic excipients. Examples of suitable excipients include lactose, mize starch or derivatives thereof, talc or stearic acid or salts thereof. Suitable excipients for use with soft gelatine capsules include, for example, vegetable oils, waxes, fats and semi-solid or liquid polyols.

For the preparation of solutions and syrups, excipients include, for example, water, polyols, saccharose, invert sugar and glucose.

Administration may also be carried out rectally, for example using suppositories, locally or percutaneously, for example using ointments, creams, gels or solution, or parenterally, for example using injectable solutions. For suppositories and for local and percutaneous application, excipients include, for example, natural or hardened oils, waxes, fats and semi-solid or liquid polyols.

The pharmaceutical compositions as described herein may also contain preserving agents, solubilizing agents, stabilizing agents, wetting agents, emulsifiers, sweeteners, colorants, odorants, buffers, coating agents and/or antioxidants.

### Dosage forms

The compound for use as described herein may be administered to the human subject in a therapeutically effective amount. As used herein, the term "therapeutically effective amount" is intended to mean the dose of compound that is sufficient to produce a therapeutic effect, for example, the quantity or dose required to eradicate or at least alleviate the symptoms associated with FOP. An appropriate amount or dose can be determined by a physician, as required. For example, the dose can be adjusted based on factors such as the size or weight of a subject to be treated, the age of the subject to be treated, the general physical condition of the subject to be treated, and the route of administration.

In some embodiments, the compound for use in accordance with the invention is administered at a dose in the range of from 0.1 mg/kg to 100 mg/kg, or from 0.25 mg/kg to 50 mg/kg. In such embodiments, the dose refers to the amount of the active ingredient administered to the subject per single administration.

In some embodiments, the compound for use as described herein is administered once daily. In alternative embodiments, the compound is administered twice daily.

In some embodiments, the compound is administered at a daily dosage in the range of from 0.2 mg/kg/day to 100 mg/kg/day, or from 0.5 mg/kg/day to 50 mg/kg/day. That is, the total amount of compound administered to the subject in one day is in the range of from 0.2 to 100 mg/kg, or from 0.5 to 50 mg/kg. In such embodiments, the compound may be administered once or multiple times per day as described herein, provided the total daily dosage is in the indicated range.

Typically, FOP patients experience episodic flare-ups, which initiate further ectopic bone formation. When palovarotene is used as a FOP therapy, higher doses of palovarotene are typically administered during these flare-ups (and sometimes for a period thereafter), with lower doses administered when the patient is not experiencing flare-ups in view of palovarotene's toxicity. In contrast, the compound for use in accordance with the present invention may be administered at a constant dosage throughout the course of the treatment (i.e. including outside of any flare-ups experienced by the subject). This is possible in view of the lower toxicity elicited by the compound compared with palovarotene.

### Additional therapeutic agents

In some embodiments, the compound for use as described herein may be administered in combination with one or more additional therapeutic agents. The one or more additional therapeutic agents may be co-formulated with compound 1 or may be formulated separately. For embodiments wherein compound 1 and the one or more additional therapeutic agents are formulated separately, administration to the subject may be via simultaneous or sequential coadministration.

### Examples

The present invention will now be described in relation to the following non-limiting examples.

### Example 1. Preparation of (E)-3-(2-(3-(tert-butyl)phenyl)-1H-indol-5-yl)acrylic acid (compound 1).

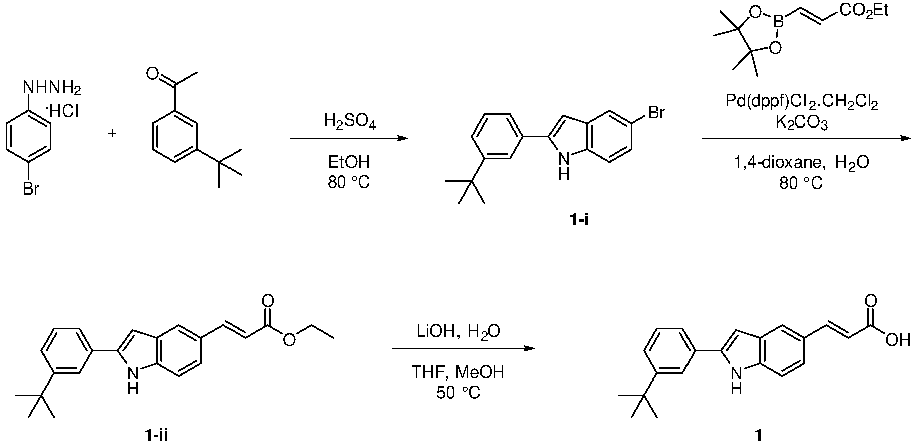

### 5-bromo-2-(3-(tert-butyl)phenyl)-1H-indole (1-i)

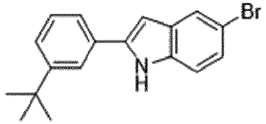

A solution of 1-(3-(tert-butyl)phenyl)ethan-1-one (1.0 eq) and (4-bromophenyl)hydrazine hydrochloride (1.1 eq) in ethanol (EtOH, 1 M) was stirred at 80 °C. After 2 h, the mixture was cooled to 5 °C and then slowly treated with an equal volume of sulfuric acid. The resulting mixture was stirred at 75 °C for 16 h and then poured into cold (5 °C) water. The aqueous suspension was extracted with ethyl acetate (3x), dried over magnesium sulfate, filtered and concentrated. The crude product was purified via automated flash chromatography using dichloromethane/hexanes as the mobile phase to afford the titled compound 1-i in 70-90% yield. ¹H NMR (400 MHz, DMSO) δ 11.76 (s, 1H), 7.88 (t, J = 1.8 Hz, 1H), 7.71 (d, J = 1.9 Hz, 1H), 7.68 - 7.63 (m, 1H), 7.44 - 7.33 (m, 3H), 7.21 (dd, J = 8.6, 2.0 Hz, 1H), 6.91 (q, J = 0.8 Hz, 1H), 1.36 (s, 9H). *m*/*z* calcd. for C₁₈H₁₈BrN = 327.1. Found [M+H]⁺ = 328.1.

### ethyl (E)-3-(2-(3-(tert-butyl)phenyl)-1H-indol-5-yl)acrylate (1-ii)

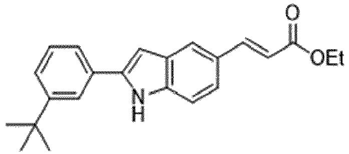

A mixture of aryl halide 1-i (1.0 eq), ethyl (E)-3-(4,4,5,5-tetramethyl-1,3,2-dioxaborolan-2-yl)acrylate (1.5 eq), 1,1'-bis(diphenylphosphino)ferrocene-palladium(ii)dichloride dichloromethane (Pd(dppf)Cl₂.CH₂Cl₂, 10 mol %), potassium carbonate (3.0 eq), 1,4-dioxane and water under a nitrogen atmosphere was stirred at 80 °C for 16 h. The mixture was cooled to ambient temperature, diluted with ethyl acetate, washed with water (3x), dried over magnesium sulfate, filtered and concentrated. The crude product was purified via automated flash chromatography using ethyl acetate/hexanes as the mobile phase to afford the titled compound 1-ii in 50-70% yield. ¹H NMR (400 MHz, DMSO) δ 11.81 (d, J = 2.2 Hz, 1H), 7.89 (dt, J = 7.1, 1.4 Hz, 2H), 7.76 (d, J = 15.9 Hz, 1H), 7.69 (dt, J = 7.1, 1.8 Hz, 1H), 7.52 (dd, J = 8.6, 1.6 Hz, 1H), 7.47 - 7.33 (m, 3H), 6.98 (dd, J = 2.2, 0.9 Hz, 1H), 6.51 (d, J = 15.9 Hz, 1H), 4.19 (q, J = 7.1 Hz, 2H), 1.36 (s, 9H), 1.27 (t, J = 7.1 Hz, 3H). *m*/*z* calcd. for C₂₃H₂₅NO₂ = 347.2. Found [M+Na]⁺ = 370.1.

### (E)-3-(2-(3-(tert-butyl)phenyl)-1H-indol-5-yl)acrylic acid (compound 1)

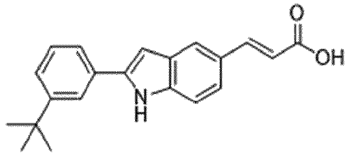

To a stirring mixture of ester 1-ii (1.0 eq) in tetrahydrofuran (THF), methanol (MeOH) and water was added lithium hydroxide (LiOH, 10 eq) and then stirred at 50 °C for 1-4 h. The mixture was cooled down to ambient temperature, quenched with aqueous 1M hydrochloric acid, extracted with ethyl acetate (3x), washed with brine, dried over magnesium sulfate, filtered and concentrated. The crude product was purified via automated flash chromatography using acetonitrile/water (0.1% formic acid) as the mobile phase to afford the titled compound 1 in 50-90% yield. ¹H NMR (400 MHz, DMSO) δ 12.18 (s, 1H), 11.79 (d, J = 2.2 Hz, 1H), 7.92-7.77 (m, 2H), 7.74 - 7.65 (m, 2H), 7.54 - 7.31 (m, 4H), 6.97 (d, J = 2.1 Hz, 1H), 6.42 (d, J = 15.9 Hz, 1H), 1.36 (s, 9H). *m*/*z* calcd. for C₂₁H₂₁NO₂ = 319.2. Found [M+H]⁺ = 320.1.

### Example 2. HeLa cell-based inhibitor assay

CYP26A1 or CYP26B1 cDNA stably transfected HeLa cells were maintained in Minimum Essential Medium (MEM) containing 10% fetal bovine serum (FBS), 1X antibiotic/antimycotic cocktail, and 100pg/mL hygromycin. Exponentially growing cells were harvested by incubation in trypsin. Cells were then collected and re-plated in 48-well culture plates at 1.5×10⁵ (CYP26A1) or 1.5×10⁴ (CYP26B1) cells in 0.2 mL of culture medium containing 0.04µCi [³H]-RA (retinoic acid) in the presence or absence of increasing concentrations of a test compound. The test compound was diluted in dimethyl sulfoxide (DMSO) and then added in duplicate to wells to 0.01 nM, 0.1 nM, 1 nM, 10 nM, 100 nM, 1 µM, and 10 µM final concentrations. Cells were incubated for 3 hours at 37 °C. Retinoids were then extracted using the procedure of Bligh, et al., (1959), Canadian Journal of Biochemistry 37, 911-917, which was modified by using dichloromethane instead of chloroform. Each sample's water-soluble radioactivity level was quantified using a β-scintillation counter (using Ecolume scintillation fluid, available from MP Biomedicals of Solon, OH, USA). IC₅₀ values represented the concentration of inhibitor required to inhibit all trans-RA metabolism by 50%, and were derived from log transformed data. Using this assay, the IC₅₀ for compound 1 generated from the dose-response curve shown in **Figure 1** was calculated to be 12.7 nM in CYP26B1 cells. In CYP26A1 cells, using this assay the percentage inhibition at the highest concentration of compound 1 tested (10 µM) was found to be 33% (see **Figure 2**).

### Example 3. Microsome-based inhibitor assay

This assay used cell-free microsomes to measure the ability of a test compound to inhibit the metabolism of RA. Microsomal fractions were obtained from CYP26B1 cDNA stably transfected HeLa cells by differential centrifugation of cell lysates. Assays were carried out in 48-well plates containing 5µg of microsomal fraction in Microsome Assay Buffer, containing 0.04µCi [³H]-RA in the presence of increasing concentrations of a test compound, as described above for the HeLa cell-based assay. Reactions were initiated by addition of 1mM NADPH, and carried out for 1 hour at 37°C. Reactions were stopped, retinoids extracted, and data were collected and analyzed as described above for the HeLa cell-based assay. Using this assay, the IC₅₀ for compound 1 generated from the dose-response curve shown in **Figure** 3 was calculated to be 5.9 nM.

### Example 4. U2OS cell-based inhibitor assay

This assay used a human cell type endogenously expressing the CYP26B1 enzyme to test the ability of a compound to block RA metabolism. U2OS cells were grown in McCoy's 5A medium containing 10% fetal bovine serum (FBS) and 1X antibiotic/antimycotic cocktail. Cells grown in a T-175 culture flask at -80% confluency were treated with 1 µM RA for 24 hours to induce the expression of the *CYP26B1* gene prior to the assay. Following this treatment period, cells were collected by trypsinization, washed twice in PBS, and resuspended in 7 ml of fresh culture medium. These cells were then re-plated in 48-well plates, and the assay was carried out as described for the HeLa-cell based assay. Using this assay, the IC₅₀ for compound 1 generated from the dose-response curve shown in **Figure 4** was calculated to be 1.9 nM.

### Example 5. RAR transcriptional activation assay

This retinoid transcriptional activity assay measures the ability of a test compound to induce expression of a RA-sensitive reporter construct. This assay utilizes MCF-7 cells stably transduced with a gene construct comprising 4 tandem RA-response elements linked to a promoter driving transcriptional expression of the firefly luciferase encoding mRNA (RARE-luc). This construct serves as a sensitive reporter of retinoid-like activity.

RARE-luc transduced MCF-7 cells were maintained in a RPMI-1640 medium containing 10% FBS. Cells were grown in 96-well plates, and treated with the test compound diluted in DMSO in duplicate at 0.1 nM, 1 nM, 10 nM, 100 nM, 1 µM and 10 µM final concentrations. As a positive control for reporter activation, cells were also treated with RA diluted in DMSO at the same concentrations. DMSO treatment alone served as a negative control. After 24 hours of treatment, cells were harvested lysis buffer (Molecular Devices, San Jose, USA) and luciferase activity in cell lysates were read using a luminometer. Luciferase data for compound 1 is shown in **Figure 5****.** As can be seen from Figure 5, most concentrations of compound 1 did not induce luciferase activity significantly, indicating that it does not directly activate the RA signaling pathway.

### Example 6. Effect of compound 1 and palovarotene on ectopic bone formation in a surgical model of heterotopic ossification

Heterotopic ossification (HO) driven by Bone Morphogenetic Protein (BMP) signaling has been used as a surrogate model for testing the effectiveness of drugs for treating Fibrodysplasia Ossificans Progressiva (FOP) (see, for example, Shimono K., et al. (2011). Nat Med 17, 454-460.). Collagen disks of 1mm in diameter were punched from a thin collagen foam matrix (Avitene^{™} Ultrafoam^{™} Collagen Hemostat product #1050050 lot #WBDP0038, from BD, Franklin Lakes, NJ, USA). Disks were impregnated with 1µg total of recombinant human BMP-2 (GenScript, Piscataway, NJ, USA, catalog #Z02913, lot #P50031712) on the day of surgery (5µl of 200ng/µL BMP-2, reconstituted in 20mM acetic acid solution). The rhBMP2-soaked collagen disk was surgically implanted into a small pocket made in the left gastrocnemius muscle of a 6-8 week-old male CD-1 mouse. Beginning on the day following surgery, mice were administered varying doses of compound 1 by oral gavage once daily (QD) or twice daily (every 12h; BID) for 13 days. Doses administered were: vehicle (Corn oil, QD (5M) and BID (3M) dosing or 3% DMSO in Corn oil (2M)), compound 1 18 mg/kg BID, 18 mg/kg QD, 6 mg/kg BID, 4.5 mg/kg BID, and 2 mg/kg BID (6M per group). Palovarotene high and low doses were used as comparators (1.76 mg/kg (4M) and 0.88 mg/kg QD (6M)). The palovarotene high and low doses were selected based on a study for palovarotene (Inubushi, T., et al. (2018). "Palovarotene Inhibits Osteochondroma Formation in a Mouse Model of Multiple Hereditary Exostoses." J Bone Miner Res 33(4): 658-666.), using oral treatment of mice. The inventors also verified the dose selection based on pharmacokinetic (PK) data generated in-house for palovarotene IP (intraperitoneal) and PO (oral) treatment by using the doses for IP treatment from Lees-Shepard, J. B., et al. (2018). "Palovarotene reduces heterotopic ossification in juvenile FOP mice but exhibits pronounced skeletal toxicity." Elife 7 to extrapolate the PO dose needed for the same exposure.

At the study endpoint (Day 14: 16-30 hours after final compound treatment) left hindlimbs were removed and submitted to micro-CT (micro-computed tomography) imaging. Using this preclinical surgical model, the amount of resulting ectopic bone formation after 14 days was visualized for each of the treatment groups (**Figure 6**). The highly enhancing regions seen within the gastrocnemius muscle corresponded to the amount of ectopic bone formation generated at the site of collagen disk implantation, following 14 days of vehicle or drug treatment. A dose-dependent decrease in ectopic bone formation was seen for compound 1 treatment, with better efficacy compared to pharmacologically relevant doses of palovarotene.

### Example 7. Comparative efficacy using a surgical mouse model of heterotopic ossification

The hindlimbs obtained in Example 6 above were submitted to micro-CT imaging. CT images were acquired using a VECTor⁴CT pre-clinical scanner (MILabs B.V., Utrecht, Netherlands) equipped with a cone beam X-ray CT system. Scans were carried out with an acceleration voltage of 50 kVp and an X-ray tube current of 430 µA in accurate focus mode to obtain 3600 projections over a 360° scan. With a 40 ms exposure time per step, the total scan procedure took about 5 minutes with an estimated radiation dose of 3077 mGy. CT images were consequently processed using MILabs reconstruction software implementing a voxel size of 20 µm and a Hann projection filter of 25 µm to generate 3-dimensional CT images. For quantification, CT images were converted to DICOM^{®} files using PMOD 3.9 software (PMOD Technologies Ltd., Zurich, Switzerland) for rendering using RadiAnt DICOM Viewer 4.2 software (Medixant, Poznań, Poland). The region-of-interest for all hindlimbs, reconstructed from DICOM files, was defined by outlining sites of ectopic ossification. Analysis of total ectopic bone volume was conducted via automated processes using the AnalyzePro software with the Bone Microarchitecture Analysis (BMA) add-on (AnalyzeDirect, Overland Park, Kansas). This software generates bone volume measurements from the defined area, as described by Bouxsein et al (2010). Journal of Bone and Mineral Research, 25(7), 1468-1486. Using this preclinical surgical model, the ability of compound 1 to inhibit HO (percentage from vehicle), as compared to palovarotene, was assessed and is shown in **Figure 7** and in Table 1 below.

**Table 1. Effect of different doses of compound 1 and palovarotene on HO in the surgical mouse model of Example 7.**

| | **Mean bone volume (mm³)** | **percent change in HO volume from vehicle mean (%)** |
|---|---|---|
| vehicle (10M) | 1.937 | **---** |
| Compound 1 18mg/kg QD (5M) | 0.436 | -77.5 |
| Compound 1 18mg/kg BID (6M) | 0.035 | -98.2 |
| Compound 1 6mg/kg BID (6M) | 0.212 | -89.1 |
| Compound 1 4.5mg/kg BID (6M) | 1.205 | -37.8 |
| Compound 1 2mg/kg BID (5M) | 0.920 | -52.5 |
| Palovarotene 1.76mg/kg QD (4M) | 0.615 | -68.2 |
| Palovarotene 0.88mg/kg (6M) | 1.985 | +2.5 |

A dose-dependent decrease in ectopic bone formation from vehicle is seen for compound 1 treatment, with better efficacy compared to pharmacologically relevant doses of palovarotene.

### Example 8. Effects on inhibition of long bone growth and growth plate microarchitecture

A preclinical study was used to evaluate toxicity of various doses of compound 1 versus palovarotene on the growth plates of juvenile FVB mice following once daily (QD) or twice (BID) administration by oral gavage, over the juvenile development period, for 4 weeks (postnatal days 14-42 (P14-P42)). Wild-type FVB animals were treated from P14-P41 with either vehicle (3% DMSO in Corn Oil QD (3M:2F), Corn Oil BID (2M:2F), 3% DMSO in Corn Oil BID (5M)), or 1.76 mg/kg palovarotene in 3% DMSO/Corn Oil QD (3M:3F), or various doses of compound 1 in corn oil (18 mg/kg QD (3M:3F), 6 mg/kg BID (3M:3F), 10 mg/kg BID (2M:3F), 3 mg/kg BID (3M:3F). Animals were weighed daily and observed for clinical signs of toxicity and other notable changes in health. At the study endpoint (postnatal day 42/study day 28) animals were submitted to µCT total body imaging, as in Example 6 above, with the following modifications: scans were performed in accurate total-body mode, and images processed using a voxel size of 60 µm and a Hann projection filter of 75 µm. Resultant DICOM files were rendered in the RadiAnt viewer to obtain long bone measurements using the program's measuring tool (**Figure 8A**). These data were normalized for differences in bone lengths inherent to variations in sex and age through transformation to a modified crural index (ratio of tibia over femur length, **Figure 8B**) (see Davenport, C. B. (1933). "The Crural Index." American Journal of Physical Anthropology 17(3): 333-353.) (Table 2). A higher modified crural index is indicative of increased toxicity.

**Table 2. Effect of different doses of compound 1 and palovarotene on mean femur length, mean tibia length and mean modified crural index.**

| **Treatment group** | **Mean femur length (cm)** | **Mean tibia length (cm)** | **Mean modified Crural index** |
|---|---|---|---|
| Vehicle (n=14) | 1.41 ± 0.04 | 1.71 ± 0.07 | 1.22 ± 0.03 |
| Compound 1 3mg/kg BID (n=6) | 1.38 ± 0.03 | 1.69 ± 0.04 | 1.23 ± 0.03 |
| Compound 1 6mg/kg BID (n=6) | 1.33 ± 0.11 | 1.53 ± 0.05 | 1.15 ± 0.05 |
| Compound 1 10mg/kg BID (n=5) | 1.34 ± 0.06 | 1.70 ± 0.04 | 1.27 ± 0.06 |
| Compound 1 18mg/kg QD (n=6) | 1.10 ± 0.09 | 1.54 ± 0.07 | 1.40 ± 0.06 |
| 1.76mg/kg QD Palovarotene (n=5) | 0.98 ± 0.04 | 1.41 ± 0.01 | 1.43 ± 0.04 |

For analysis of growth plate histology, right tibiofemoral joints were dissected post-mortem and processed into formalin overnight. Joints were transferred into Osteosoft^{®} or EDTA mild decalcifying agents (both available from Sigma, Oakville, ON, Canada) for 48-72h before being stored in 70% ethanol. Samples were then processed into paraffin wax for sectioning onto glass slides. Slides were stained using standard protocols for Safranin O with Fast Green counterstain to examine cartilage morphology. Using this model, the comparative growth effect on long bones (tibia and femur, **Figure 8A***;* modified crural index **Figure 8B**) and on the histological microarchitecture of the developing growth plates (**Figure 9**) were obtained for efficacious doses of compound 1 as compared to palovarotene. These data show that the disturbances to the growth of long bones and microarchitecture of the growth plates that are typical of palovarotene treatment are not observed for compound 1 at doses which are at least equally efficacious as palovarotene.

### Example 9. Mapping a therapeutic window for compound 1 and palovarotene

A 50% reduction in progression of ectopic bone formation has been suggested to be a metric that is the minimum tolerated efficacy clinically relevant for FOP patients, by expert consensus (Smilde BJ, et al. (2022) BMC Musculoskelet Disord. Jun 1 ;23(1):519). For both palovarotene and compound 1 efficacy and toxicity were correlated to the pharmacokinetic parameters AUC (area under the curve), Cmax (maximum concentration detected following compound administration), and duration above EC50/IC50.This illustrated that efficacy was best predicted by duration above the EC50/IC50 (effective concentration for 50% response (in the case of palovarotene), or for 50% inhibition (in the case of compound 1)), whereas toxicity was best predicted by Cmax. The surgical efficacy studies outlined in Example 7 above, along with the effects on growth plates outlined in Example 8 (where "toxicity" is defined here as a greater than 5% increase in modified crural index from control), were used to plot the therapeutic window between the effective dose for 50% reduction in ectopic bone (ED50) and the maximum tolerated dose (MTD) for toxicity as defined above. For palovarotene, efficacy data from Example 7 and toxicity data from Example 8 above were compiled with mined data from the following publications:
Lindborg et al (2018). "Cartilage-derived retinoic acid-sensitive protein (CD-RAP): A stage-specific biomarker of heterotopic endochondral ossification (HEO) in fibrodysplasia ossificans progressiva (FOP)." Bone 109: 153-157.
Chakkalakal, S. A., et al. (2016). "Palovarotene Inhibits Heterotopic Ossification and Maintains Limb Mobility and Growth in Mice With the Human ACVR1(R206H) Fibrodysplasia Ossificans Progressiva (FOP) Mutation." J Bone Miner Res 31(9): 1666-1675.
Inubushi, T., et al. (2018). "Palovarotene Inhibits Osteochondroma Formation in a Mouse Model of Multiple Hereditary Exostoses." J Bone Miner Res 33(4): 658-666.
Lees-Shepard, J. B., et al. (2018). "Palovarotene reduces heterotopic ossification in juvenile FOP mice but exhibits pronounced skeletal toxicity." Elife 7.
Shimono, K., et al. (2011). "Potent inhibition of heterotopic ossification by nuclear retinoic acid receptor-gamma agonists." Nat Med 17(4): 454-460.

**Figure 10** shows that compound 1 has a therapeutic window between 50% efficacy (MED) and toxicity (MTD) of at least 2-fold, whereas there is no dose of palovarotene which is able to elicit efficacy of 50% or greater, without toxicity. In particular, compound 1 has a ED50 of 4 mg/kg BID and a MTD of 10 mg/kg BID, while palovarotene has a ED50 of 0.3 mg/kg QD and a MTD of 0.285 mg/kg QD.

### Example 10. Efficacy in a genetic model of FOP

*Acvr1*^{*(cR206H*/*+)*} mice were obtained from the International Fibrodysplasia Ossificans Progressiva Association (IFOPA, North Kansas City, MO, USA). These animals harbour a Cre recombinase-inducible *Acvr1* transgenic allele, which causes a switch from wild-type mouse ACVR1 protein expression to that of a chimeric mouse/human protein containing the R206H mutation seen in >95% of FOP patients. These animals were intercrossed with *Pdgfra-Cre* expressing mice (C57BL/6-Tg(PDGFRA-cre)1Clc/J mice, available through The Jackson Laboratory, Bar Harbor, ME, USA) which activates Cre recombination in fibro-adipogenic precursors, currently believed to be the cells of origin for FOP (Lees-Shepard, J.B., et al (2018) Nat Commun 9, 471.). Resulting study animals (ACVR1^{(R206H/+)}*;Pdgfra*-Cre+) are susceptible to injury-induced ectopic bone formation. These animals were subjected to a bilateral gastrocnemius pinch procedure under anaesthesia, whereby a Halsted-mosquito hemostat (product#13008-12, Fine Science Tools Inc., North Vancouver, BC, Canada) is used to compress the gastrocnemius muscle, at its widest part, to approximately 2mm for 5s, and then released. This injury elicits robust ectopic bone formation within 10-14 days (for description of procedure see, Lyu, H., et al. (2021). Biomedicines 9(6).). Animals began treatment with various doses of compound 1 (2.5 mg/kg BID, 4 mg/kg BID, 7.5 mg/kg BID and 10 mg/kg BID) or corn oil vehicle on the day following the procedure for 21 days. Micro-CT imaging was performed on days 14 (under anaesthesia) and at study termination (day 22), as outlined above in Example 7. Sites of ectopic bone formation in the area of the injury were volumetrically quantified using Class stratification in Imalytics Preclinical software (version 3.0.2.3, Gremse-IT GmbH, Aachen, Germany) from DICOM renderings. Using this model, the utility of compound 1 in inhibiting bone formation in a genetic mouse model of FOP was illustrated (see **Figure 11** and Table 3 below).

**Table 3. Effect of different doses of compound 1 on mean ectopic bone volume at 14 and 22 days following initiation of treatment**

| **Treatment group** | **Mean ectopic bone volume D14 (mm³) [percent change from vehicle]** | **Mean ectopic bone volume D22 (mm³) [percent change from vehicle]** |
|---|---|---|
| Vehicle (n=12) | 12.63 ± 8.61 | 11.85 ± 10.99 |
| Compound 1 10mg/kg BID (n=12) | 1.28 ± 2.23 [-89.9%] | 1.40 ± 2.65 [-88.2%] |
| Compound 1 7.5mg/kg BID (n=10) | 0.31 ± 0.45 [-97.5%] | 0.07 ± 0.18 [-99.4%] |
| Compound 1 4mg/kg BID (n=6) | 0.20 ± 0.47 [-98.5%] | 0.00 ± 0.00 [-100%] |
| Compound 1 2mg/kg BID (n=8) | 2.92 ± 3.76 [-76.9%] | 2.62 ± 4.36 [-77.9%] |

The foregoing detailed description has been provided by way of explanation and illustration and is not intended to limit the scope of the appended claims. Many variations in the presently preferred embodiments illustrated herein will be apparent to one of ordinary skill in the art and remain within the scope of the appended claims and their equivalents.

Various publications are cited in the foregoing description, each of which is incorporated by reference in its entirety.

## Claims

1. A compound for use in treating fibrodysplasia ossificans progressiva (FOP) in a human subject, wherein the compound is in accordance with the following formula: 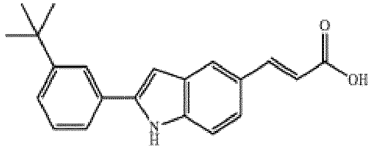 or a pharmaceutically acceptable salt thereof.

2. The compound for use according to claim 1, wherein the human subject has a R206H mutation in the intracellular glycine-serine domain of human ACVR1.

3. The compound for use according to claim 1 or claim 2, wherein the human subject is a pediatric subject.

4. The compound for use according to claim 3, wherein the pediatric subject is a male having an age of 9 years or less or a female having an age of 7 years or less.

5. The compound for use according to any of the preceding claims, wherein treating FOP comprises decreasing or preventing the formation of heterotopic ossification lesions in the subject.

6. The compound for use according to claim 5, wherein the heterotopic ossification lesions comprise calcified soft tissue.

7. The compound for use according to any of the preceding claims, wherein the compound is administered orally.

8. The compound for use according to any of the preceding claims, wherein the compound is administered at a daily dosage in the range of from 0.2 mg/kg/day to 100 mg/kg/day.

9. The compound for use according to any of the preceding claims, wherein the compound is administered once daily or twice daily.

10. The compound for use according to any of the preceding claims, wherein the compound is formulated as a pharmaceutical composition additionally comprising one or more pharmaceutically acceptable diluents, excipients or carriers.
